(19)

Europäisches
Patentamt
European
Patent Office
Office européen
des brevets

(11) **EP 1 998 668 B1**

(12) **EUROPEAN PATENT SPECIFICATION**

(45) Date of publication and mention
of the grant of the patent:
**23.05.2012 Bulletin 2012/21**

(21) Application number: **06828018.9**

(22) Date of filing: **18.12.2006**

(51) Int Cl.:
***A61B 5/04*** (2006.01)

(86) International application number:
**PCT/AU2006/001907**

(87) International publication number:
**WO 2007/076566 (12.07.2007 Gazette 2007/28)**

(54) **METHOD AND APPARATUS FOR LOCALISING AND DISPLAYING ELECTROPHYSIOLOGICAL SIGNALS**

VERFAHREN UND VORRICHTUNG ZUR LOKALISATION UND ANZEIGE VON ELEKTROPHYSIOLOGISCHEN SIGNALEN

PROCEDE ET APPAREIL POUR LOCALISER ET AFFICHER DES SIGNAUX ELECTROPHYSIOLOGIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.01.2006 AU 2006900045**

(43) Date of publication of application:
**10.12.2008 Bulletin 2008/50**

(73) Proprietor: **Compumedics Limited
Abbotsford, Victoria 3067 (AU)**

(72) Inventor: **WAGNER, Michael
20357 Hamburg (DE)**

(74) Representative: **Hands, Lewis Roger et al
Handsome I.P. Ltd
3 Chapel Row
Bath, North East Somerset BA1 1HN (GB)**

(56) References cited:
**US-A1- 2003 093 004**

- **YONGXIA ZHOU ET AL: "Spatiotemporal source localization with new reweighted method: a simulation study", 2003 IEEE NUCLEAR SCIENCE SYMPOSIUM CONFERENCE RECORD. / 2003 IEEE NUCLEAR SCIENCE SYMPOSIUM AND MEDICAL IMAGING CONFERENCE. PORTLAND, OR, OCT. 19 - 25, 2003; [IEEE NUCLEAR SCIENCE SYMPOSIUM CONFERENCE RECORD], NEW YORK, NY : IEEE, US, vol. 4, 19 October 2003 (2003-10-19), pages 2776-2779, XP010737617, ISBN: 978-0-7803-8257-2**
- **PASCUAL-MARQUI R.D.: 'Standardized low resolution brain electromagnetic tomography (sLORETA): technical details' METHODS & FINDINGS IN EXPERIMENTAL & CLINICAL PHARMACOLOGY vol. 24D, 2002, pages 5 - 12, XP003015350**
- **LIN ET AL.: 'Assessing and improving the spatial accuracy in MEG source localization by depth-weighted minimum-norm estimates' NEUROIMAGE vol. 31, 2006, pages 160 - 171, XP005439555**
- **DAUNIZEAU ET AL.: 'Bayesian Spatio-Temporal Approach for EEG Source Reconstruction: Conciliating ECD and Distributed Models' IEEE TRANSACTIONS ON BIOMEDICAL ENGINEERING vol. 53, no. 3, March 2006, pages 503 - 516, XP003015351**
- **LIU ET AL.: 'Standardized Shrinking LORETA-FOCUSS (SSLOFO): A New Algorithm for Spatio-Temporal EEG Source Reconstruction' IEEE TRANSACTIONS ON BIOMEDICAL ENGINEERING vol. 52, no. 10, October 2005, pages 1681 - 1691, XP003015352**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Field of the Invention

The present invention relates to apparatus for acquiring electrophysiological signals associated with physiological processes, in particular, electroencephalogram (EEG) and magnetoencephalogram (MEG) measurements, and to methods for analysis of electrical signals produced in said measurements by said apparatus.

## Background

Brain activity can be represented by data from EEGs and MEGs, which are comprised of measurements of electrical signals from electrode sensors positioned adjacent a head (EEG) or coils positioned above (MEG) the surface of a head. In the analysis of acquired EEG and MEG data from sensor outputs, brain activity can be represented as a discrete three-dimensional vector field, each vector denoting a dipolar electrical current source, hereinafter referred to as a "current source". The result provides a representation of the underlying synaptic activity of neurons in the working brain at a point in time and over time.

It is known in the art that the relationship between the aforementioned vector field and the measured electrical signals is linear. The relationship is uniquely determined by the sensor layout adjacent (EEG) or above the head (MEG), the choice of reference (ground), the measurement noise, and the electrical conducting properties of the head, known as a "forward model". For any time point, this linear relationship can be written as $\mathbf{A}\,\mathbf{x} + \mathbf{n} = \mathbf{b}$, where $\mathbf{A}$ (the lead field matrix) represents the forward model and the choice of reference, $\mathbf{n}$ represents the measurement noise, $\mathbf{b}$ represents the measured data, and $\mathbf{x}$ represents the vector field of electrical current sources, "the currents", comprising three entries per discretization point. For convenience, in this document, symbols representing matrices are written in bold uppercase; symbols representing vectors are written in bold lowercase; and the $N^{th}$ entry of a vector, $\mathbf{x}$, is identified by $x_N$ and the $(M,N)^{th}$ entry of a matrix, $\mathbf{A}$, is identified by $A_{M,N}$.

For a particular distribution of current sources, the sensor layout, the reference, the forward model and, assuming no electrical noise, the measured physiological electric signal data can be predicted uniquely. This is known as the "forward problem". However, there is the problem that, for any chosen set of measured physiological electrical signal data, the sensor layout, the reference, and the forward model, the distribution of current sources cannot be computed uniquely due to any of the following reasons:

- the number of sensors is limited; or
- the noise is unknown; or
- there are typically more unknown values (currents) than there are known values (sensors); or
- there exist current configurations (silent sources) that produce no measurable signal.

Such a problematic situation, which is common with electrophysiological measurements, is known in the art as an ill-posed, ill-conditioned inverse problem. However, the estimation of these currents is an important objective in EEG and MEG analysis, for example, to make the output of the measurements meaningful.

Methods known in the art for computing the currents, given the measured physiological signal data, utilize a data model comprising the noise characteristics of the data and a source model comprising assumed features of the currents. The noise characteristics of the data (data model) are typically expressed using a noise covariance matrix, $\mathbf{C_n}$, which can be estimated from the measured signal data using said assumptions. Data and the lead field matrix can also be "pre-whitened", yielding a noise covariance matrix of $\mathbf{1}$.

A widely made assumption in the art regarding the characteristics of the currents (source model) is that most currents are small or zero. This assumption derives, for example, from the nature of an observed brain state where one localized type of activity might be assumed to dominate, or by the nature of an experiment, where many instances of data sharing a common feature of interest are averaged and, consequently, all but the observed feature is assumed to be suppressed by the averaging process. A corresponding source model is the minimum-norm least-squares model, where the L2-norm $\mathbf{x}^T \mathbf{C_s}^{-1} \mathbf{x}$ is assumed to be minimal, with $C_s$ being the source covariances of $\mathbf{x}$. If no information regarding the source covariances is available, then $\mathbf{C_s} = \mathbf{1}$. Regularization is used to balance the influence of the data model and the source model. Following this line of reasoning, a unique $\mathbf{x_{opt}}$, (where $\mathbf{x}_{opt}$ is the optimal vector field as defined above) can be obtained by solving a linear inverse problem by minimization of the expression,

$$\mathbf{x_{opt}} = \arg\min \left[ (\mathbf{A}\,\mathbf{x} - \mathbf{b})^T \mathbf{C_n}^{-1} (\mathbf{A}\,\mathbf{x} - \mathbf{b}) + \lambda\, \mathbf{x}^T \mathbf{C_s}^{-1} \mathbf{x} \right],$$

where $\lambda$ is the regularization parameter. It is well-known in the art that an analytical solution for $\mathbf{x}_{opt}$ can be obtained. Furthermore, it is well-known in the art that an optimal value of $\lambda$ can be obtained based on no additional information.

It is known in the art that for linear inverse problems, such as the one presented here for the data derived from electrophysiological signal measurements of the source and type described herein, the laws of additivity and superposition apply and that it follows from these laws that the localization power of a linear inverse method can be characterized by its ability to localize point sources. The solution, $\mathbf{x}_{opt,P}$, that is obtained for synthetic data, $\mathbf{b} = \mathbf{A}\,\mathbf{x}_P$, where $x_P = 1$ and all other entries in $\mathbf{x}$ are zero, is called the point spread function of the $P^{th}$ current source. A method localizes point sources exactly if and only if, for all values of P, $(x_{opt,P})_P$ is the single largest entry of $\mathbf{x}_{opt,P}$. According to this criterion, the current methods in the art are not capable of exactly localizing point sources. What is needed is a method and apparatus for collecting and transforming electrophysiological signal data that have the characteristics of point sources to provide useful and improved representations of electrical activity for physiological analysis.

LIU ET AL.: "Standardized Shrinking LORETA-FOCUSS (SSLOFO): A New Algorithm for Spatio-Temporal EEG Source Reconstruction", IEEE TRANSACTIONS ON BIOMEDICAL ENGINEERING, vol. 52, no.10, October 2005, pages 1681-1691, XP003015352 discloses solving a weighted linear inverse problem to get a source current density vector field estimation, computing a diagonal weighting matrix in which entries are determined by the source current density vector field estimation, and solving a weighted linear inverse problem to get a source power.

YONGXIA ZHOU ET AL: "Spatiotemporal source localization with new reweighted method: a simulation study", 2003 IEEE NUCLEAR SCIENCE SYMPOSIUM CONFERENCE RECORD. /2003 IEEE NUCLEAR SCIENCE SYMPOSIUM AND MEDICAL IMAGING CONFERENCE. PORTLAND, OR, OCT. 19 - 25, 2003; [IEEE NUCLEAR SCIENCE SYMPOSIUM CONFERENCE RECORD], NEW YORK, NY : IEEE, US, vol. 4, 19 October 2003, pages 2776-2779, XP01 0737617, ISBN: 978-0-7803-8257-2 discloses using only the maximum absolute amplitude for each source of the time series of the spatio-temporal dipole model based method for re-weighting.

In this document, the words, "including" or "comprising", are used interchangeably with the same meaning, which is to be not limited to any stated feature or list of features.

## Summary of the Invention

It is an object of the invention to provide an improved method of transforming data comprising EEG and/or MEG signal measurements to represent brain activity. It is a further object to provide an algorithm that can be implemented in computer software to analyse electrophysiological signal data to provide a result comprising of a representation of physiological activity for interpretation and analysis. It is a further object to provide an apparatus to acquire physiological signal measurements with a linear relationship as described herein and transform the signal measurements into representations of physiological activity.

The invention provides a method for analysis of electrophysiological signal data to enable physiological interpretation of measured signals. According to the invention a discrete source current vector field, $\mathbf{x}_{opt}$, from measured physiological signal data, $\mathbf{b}$, and a lead field matrix, $\mathbf{A}$, are computed. For point sources, the current density vector field, $\mathbf{x}_{opt}$, has its maximum at the location of the point source.

In one aspect, the invention provides a method for transforming electrical signal data from sensors using a microprocessor, including the steps of collecting and storing electrical signal data into a computer file; computing a current density vector field by solving the related unweighted linear inverse problem; processing the current density vector field using an sLORETA transformation; computing a diagonal weighting matrix so that its entries are determined by a monotonically increasing function of their corresponding values in the sLORETA method outputs; computing the current density vector field by solving the related weighted linear inverse problem; and storing the resulting data in at least one computer file. Preferably the method includes the steps of applying a data imaging technique to the stored resulting data for transforming the data into a form suitable for visual representation of the data and displaying the transformed data for visual inspection.

In another aspect, the invention provides apparatus for collecting, transforming and displaying electrical signal data, comprising: sensors for collecting electrical signals; means for storage of electrical signal data; and at least one microprocessor having a computer program configured to implement the above method steps.

Preferably the apparatus includes means for storing transformed data. Preferably the apparatus includes means for displaying the transformed data.

## Brief Description of the Figures

Figure 1 shows a flowchart of the method of the invention.

Figure 2 shows an example of an EEG with electrical impulses measured on 28 channels in Fig 2a and a computer-generated 3D representation in Fig 2b. The outcome of Step 1 is shown here.

Figure 3 shows an example of an analysis of EEG data using the method of the invention. The outcome of Step 2 is shown here.

Figure 4 shows a further example of an analysis of EEG data using the method of the invention. The outcome of Step 3 is shown here.

Figure 5 shows a further example of an analysis of EEG data using the method of the invention. The final result is shown here.

**Detailed Description of the Drawings and Best Method of Performance**

The method is most conveniently applied to signals of EEG and MEG measurements to provide a result that shows a representation of brain activity. It will be understood that the invention is most advantageously applied to the collection and analysis of EEG and MEG data, but that the method is not limited to the analysis of EEG and MEG data, the invention having more general application such as in the application to electrocorticogram (ECoG) measurements of brain activity, electrocardiogram (ECG) measurements and magnetocardiogram (MCG) measurements of heart activity, for example. The invention provides a method for analysis of data, including electrophysiological data, which displays the linear relationship described herein, or can be linearized (using, e.g., Newton's method) to do so. Other fields in which the method of the invention is useful beside the examples of medical imaging described herein where inverse problems are used include geophysics and astronomy, for example. The invention is useful in all cases where $\mathbf{x}$ is known to be sparse and the range of column norms found in matrix $\mathbf{A}$ is wide.

According to the invention, two known data-transformation techniques are used to transform collected physiological signal measurements stored as data in a computer file.

A first transformation technique, known as sLORETA, localizes point sources correctly. However, its result, s, is not a source current vector field but a statistical measure based on $\mathbf{x_{opt}}$. The sLORETA vector, s, has one entry per discrete location and is computed by dividing each location's entries of $\mathbf{x_{opt}}$ by their posterior variances. The method incorporates by reference the sLORETA technique described by Pascual-Marqui (2002), which is a post-processing step that transforms the source current vector field into a form that is better suited for representations for interpretation by a person or for input to a statistical software package for further analysis.

A second transformation technique, known as "Source Weighting" utilizes the equality,

$$\mathbf{C_s = W^{-2} C_p,}$$

where $\mathbf{C_p}$ is the source covariance matrix of $\mathbf{x}$. $\mathbf{C_p}$ encodes external prior knowledge about the source distribution. If no such information is available, $\mathbf{C_p = 1}$. The diagonal weighting matrix $\mathbf{W}$ is determined by the Source Weighting method itself. Given $\mathbf{A}$, $\mathbf{b}$, and $\mathbf{C_p}$, different values of $\mathbf{x}$ are obtained depending on $\mathbf{W}$.

The method of the invention conveniently implements the herebefore described techniques into computer software for transforming electrical signal data into representations in ways not previously thought to be useful.

Both techniques described herein are known in the art. However, the sLORETA technique is used in the art as a data post-processing technique. According to the invention the sLORETA technique is a pre-processing step that provides the surprising utility found in the result when used in combination with the Source Weighting technique. The method of the invention when used with electrophysiological signal measurements, for example, EEG or MEG measurements or other suitable measurements, has not previously been shown.

The invention includes a device having electrodes for acquiring electrophysiological signal data, a means for storing said data, a means for transforming said data, a microprocessor for making calculations in the transformation, computer software implementing the algorithm of the method, a means for storing transformed data, and a means for displaying transformed data. In one embodiment, the invention comprises an EEG apparatus and electrodes for measuring an EEG, a means for electronically storing EEG data, a means for storing computer software and executing computer software implementing the invention, a means for electronically storing transformed data and a screen for displaying transformed data. The screen may be any suitable screen capable of displaying images. This may include screens on analogue or digital monitors. It will be understood that the scope of the invention includes many embodiments that will achieve the objects.

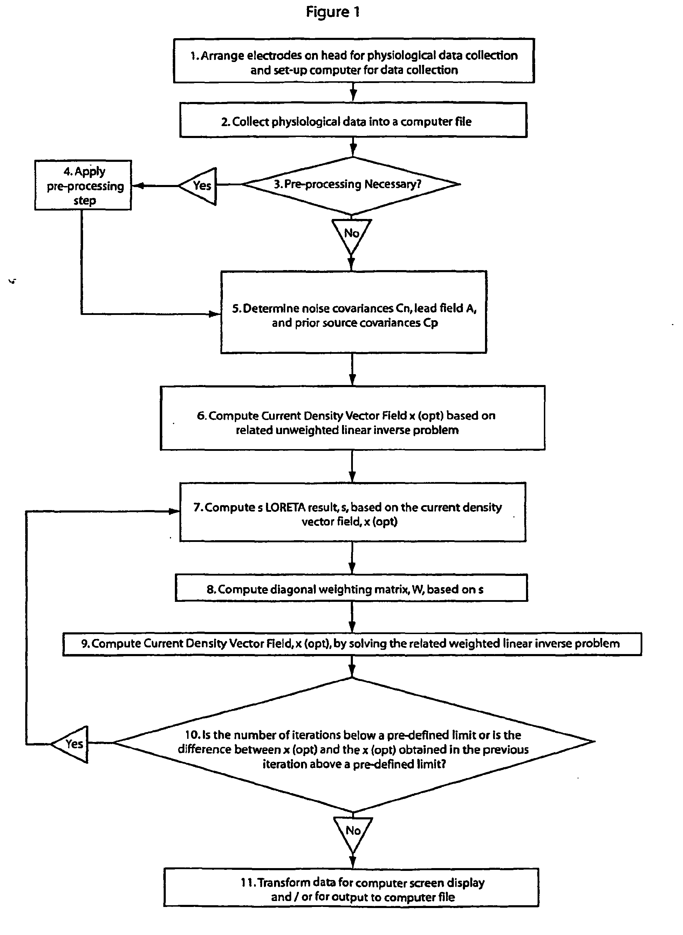

Embodiments of the method include combinations of data collection and transformation steps illustrated in the boxes in the flowchart shown in Figure 1. Initially, sensor electrodes are arranged adjacent the head of a subject, for example, in the case of EEG and MEG, and a computer is set up to collect and transform outputs into computer data files 1. It will be understood that the scope of the invention includes any type of physiological signals that are suitable for use in the method as described herein. Transformed data representing electrophysiological signals is collected and/or stored for further processing 2. In processing the data, a determination is made whether or not to pre-process the data 3. The data may be pre-processed 4 as indicated below, or the noise covariances, lead field, and prior source covariances may be calculated 5 without pre-processing. Subsequently, the current density vector field is computed, based on the related unweighted linear inverse problem 6. The sLORETA technique is applied to the current density vector field, $\mathbf{x_{opt}}$, to calculate the result, $\mathbf{s}$, 7 and then the diagonal weighting matrix, is calculated 8. The current density vector field is also computed by solving the related weighted linear inverse problem 9. If the number of iterations is below a pre-defined limit or the difference between the two values of $\mathbf{x_{opt}}$ calculated by the two techniques 10, the sLORETA result may be recalculated 7 or the resulting data may be stored in random-access memory (RAM) for further transformation by suitable data-imaging techniques for representation of the data for visual display or output to a computer file 11 for later use.

More specifically, the method using sLORETA determines the weighting matrix, $\mathbf{W}$, in the following steps:

a) Collecting electrical signal data into a computer file. Optionally, applying pre-processing such as averaging or filtering. Determine noise covariances $\mathbf{C_n}$, lead field $\mathbf{A}$ and prior source covariances $\mathbf{C_p}$

b) Computing the current density vector field $\mathbf{x_{opt}}$ by based on the measured data b, noise covariances $C_n$, lead field A and prior source covariances $\mathbf{Cp}$ by solving the related unweighted linear inverse problem

c) Computing the sLORETA result s based on the current density vector field $\mathbf{x_{opt}}$

d) Computing the diagonal weighting matrix W so that its entries (three per location) are determined by a monotonically increasing function of their corresponding values in s (one per location), e.g. $W_{3i+0,\ 3i+0} = W_{3i+1,\ 3i+1} = W_{3i+2,\ 3i+2} = S_i$

e) Computing the current density vector field $\mathbf{x_{opt}}$ based on the measured data $\mathbf{b}$, noise covariances $\mathbf{C_n}$, lead field $\mathbf{A}$, diagonal weighting matrix W and prior source covariances $\mathbf{C_p}$ by solving the related weighted linear inverse problem.

f) Optionally, iterating by repeating steps c), d), e) a fixed number of times or until successively obtained current density vector fields $\mathbf{x}_{opt}$ are equal within a predefined limit.

The method of the invention is most conveniently practised by implementing the method in a computer algorithm. In particular, there is a large amount of signal data acquired in the measurement of an EEG or MEG that must be transformed by the method of the invention to provide a meaningful result.

**Examples**

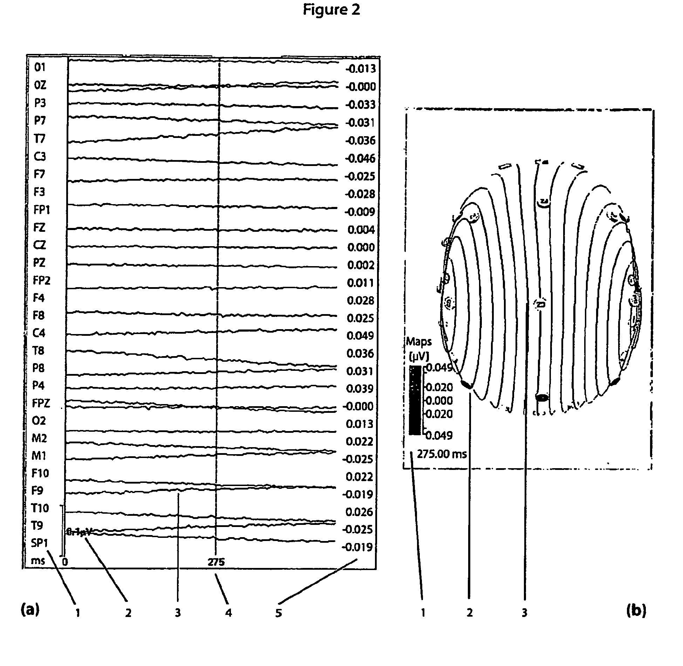

Simulated EEG data containing point sources are shown in Figure 2. In Figure 2a, on the left, the output 3 of 28 sensors located on the head in an EEG is shown, together with its scale 2 and each channel's amplitude 5 at the time point depicted by the vertical time cursor 3 which denotes the time point used for analysis. Furthermore, each sensor (channel) is labelled according to the sequence on the left-hand side 1. In Figure 2b, on the right, a computer-generated three-dimensional (3D) rendering of the sensors 2 (identified by their labels) and isopotential lines of the voltages 3 for the selected time point are shown together with the scale used 1. The noise covariance matrix $\mathbf{C_n}$ is diagonal in this example, and all its entries are $(1\ nV)^2$ which corresponds to a signal-to-noise ratio of 25. The source prior covariance matrix $\mathbf{C_p}$ is $\mathbf{1}$.

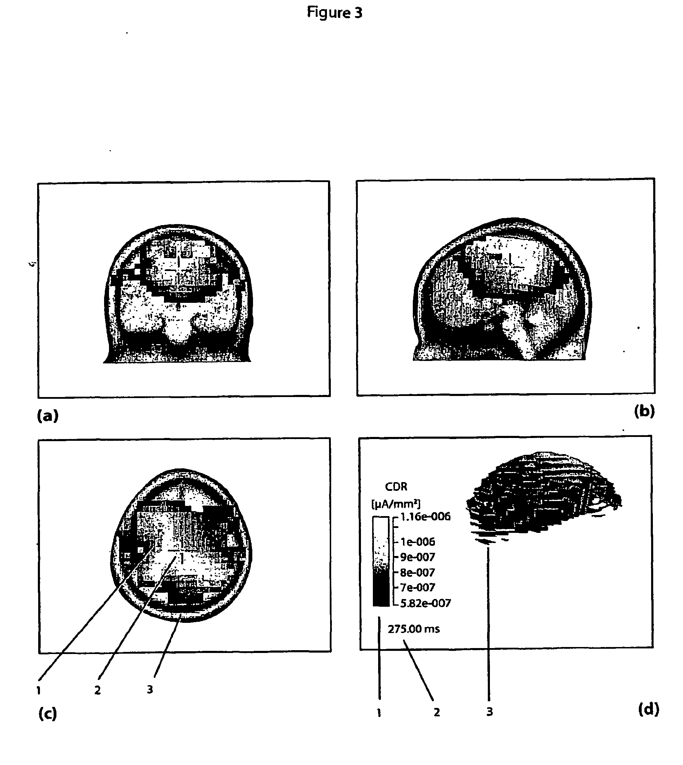

Figures 3 to 5 show analysis results applied to EEG signal data. In all of these figures, in parts a)-c), three orthogonal cuts through the 3D solution space show the analysis results 1, together with an anatomical backdrop 3 which is depicted for illustration purposes only and has no further relevance to the data displayed. In the orthogonal cuts, the black crosshair 2 shows the location of the simulated point source. In part d) a computer-generated 3D rendering can be seen. The 3D rendering shows the analysis result 3 together with its scale 1 and the time point used for analysis 2.

Figure 3 shows the results of an unweighted linear inverse solution (minimum norm least squares analysis) where $\mathbf{W}$ = 1. The units seen at the scale are $\mu A/mm^2$ which is current dipole moment $\mu Amm$ per volume (each current accounts for a cubic $(7mm)^3$ volume).

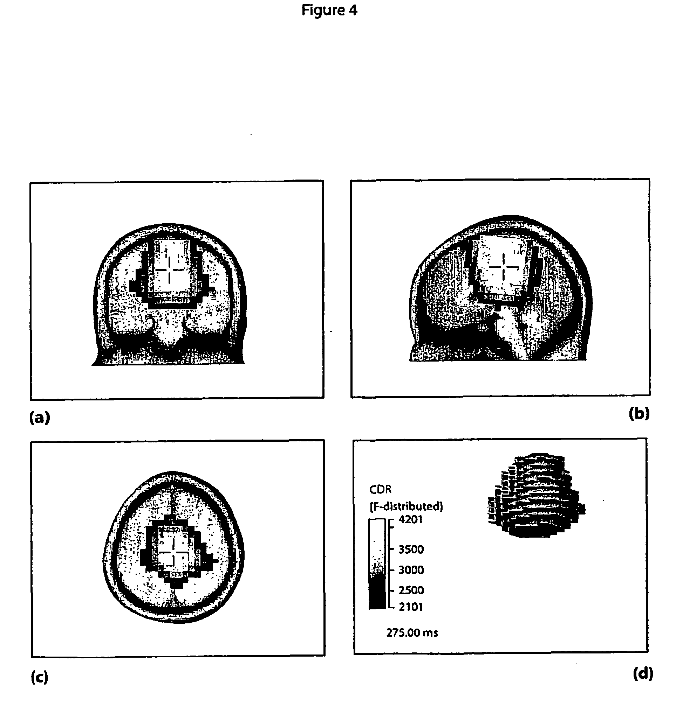

Figure 4 shows the results of an sLORETA analysis with its maximum at the crosshair. However, values depicted are not currents but F-distributed statistical scores.

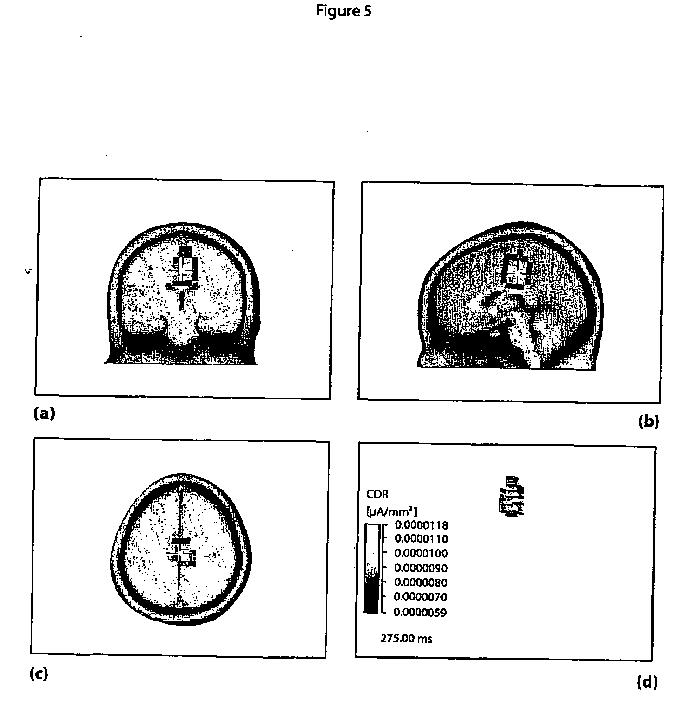

Figure 5 shows the results of the proposed method. Again, with its maximum at the crosshair. The units seen at the scale are $\mu A/mm^2$ which is current dipole moment $\mu Amm$ per volume (each current accounts for a cubic $(7mm)^3$ volume).

**References**

Dale A.M., Sereno M.I. Improved Localization of Cortical Activity by Combining EEG and MEG with MRI Cortical Surface Reconstruction: A Linear Approach. Journal of Cognitive Neuroscience 5, 162-176, 1993.

Fuchs M., Wagner M., Köhler T., Wischmann H.A. Linear and Nonlinear Current Density Reconstructions. J Clin Neurophysiol 16, 267-295, 1999.

Hämäläinen M., limonlemi R. Interpreting magnetic fields of the brain: minimum norm estimates. Report TKK-F-A559, Helsinki University of Technology, Espoo, 1984.

Köhler T. Lösungen des bioelektromagnetischen inversen Problems. PhD-Thesis, University of Hamburg, Hamburg, 1998.

Pascual-Marqui R.D. Review of methods for solving the EEG inverse problem. International Journal of Bioelectromagnetism 1, 75-86, 1999.

Pascual-Marqui R.D. Standardized low resolution brain electromagnetic tomography (sLORETA): technical details. Methods & Findings in Experimental & Clinical Pharmacology 24D, 5-12, 2002.

Pascual-Marqui R.D., Michel C.M., Lehmann D. Low resolution electromagnetic tomography: a new method for localizing electrical activity in the brain. International Journal of Psychophysiology 18, 49-65, 1994.

Sekihara K., Sahani M., Nagarajan S.S. Localization bias and spatial resolution of adaptive and non-adaptive spatial filters for MEG source reconstruction. NeuroImage 25, 1056-1067, 2005

Tarantola A. Inverse Problem Theory (2nd edition). Elsevier, Amsterdam, 1994.

Wagner M. Rekonstruktion Neuronaler Ströme. Shaker Verlag, Aachen, 1998.

Wagner M., Fuchs M., Kastner J. Evaluation of sLORETA in the presence of noise and multiple sources. Brain Topogr. 16, 277-80, 2004.

**Claims**

1. A method for transforming electrical signal data from sensors using a microprocessor, including the steps of:

   a) collecting and storing electrical signal data into a computer file (2);
   b) computing a current density vector field by solving the related unweighted linear inverse problem (6);
   c) processing the current density vector field using an sLORETA transformation (7);
   d) computing a diagonal weighting matrix so that its entries are determined by a monotonically increasing function of their corresponding values in the sLORETA method outputs (8);
   e) computing the current density vector field by solving the related weighted linear inverse problem (9); and
   f) storing the resulting data in at least one computer file (11).

2. The method of claim 1, further including the step of applying a data imaging technique to the stored resulting data for transforming the data into a form suitable for visual representation of the data (11).

3. The method of claim 2, further including the step of displaying the transformed data for visual inspection.

4. Apparatus for collecting and transforming electrical signal data, comprising:

   sensors for collecting electrical signals;
   means for storage of electrical signal data; and
   at least one microprocessor having a computer program configured to implement the steps of claim 1.

**5.** Apparatus according to claim 4 further comprising means for storing transformed data.

**6.** Apparatus according to claim 4 or claim 5 further comprising means for displaying the transformed data.

**Patentansprüche**

**1.** Eine Methode zum Umwandlung elektrischer Signaldaten von Sensoren mit Hilfe eines Mikroprozessors, die die folgenden Schritte umfasst:

a) Erfassung und Speicherung elektrischer Signaldaten in einer Datei (2);
b) Berechnung eines Stromdichte-Vektorfelds durch Lösung des in diesem Zusammenhang auftretenden nicht gewichteten linearen inversen Problems (6);
c) Verarbeitung des Stromdichte-Vektorfelds mit Hilfe einer sLORETA-Umrechnung (7);
d) Berechnung einer diagonalen Gewichtungsmatrix, so dass deren Einträge durch eine monoton steigende Funktion ihrer entsprechenden Werte innerhalb der Outputs der sLORETA-Methode (8) bestimmt werden;
e) Berechnung des Stromdichte-Vektorfelds durch Lösung des in diesem Zusammenhang auftretenden gewichteten linearen inversen Problems (9); sowie
f) Speicherung der Ergebnisdaten in mindestens einer Computerdatei (11).

**2.** Die Methode gemäß Anspruch 1, einschließlich des Schrittes der Anwendung einer Daten-Bildgebungstechnik auf die gespeicherten Ergebnisdaten, um die Daten in eine zur visuellen Darstellung der Daten (11) geeignete Form umzuwandeln.

**3.** Die Methode gemäß Anspruch 2, einschließlich des Schritts der Anzeige der umgewandelten Daten zur visuellen Prüfung.

**4.** Gerät zur Erfassung und Umwandlung elektrischer Signaldaten, das Folgendes umfasst:

Sensoren zur Erfassung elektrischer Signale;
Mittel zur Speicherung elektrischer Signaldaten; sowie mindestens einen Mikroprozessor, der über ein für die Durchführung der Schritte aus Anspruch 1 konfiguriertes Computerprogramm verfügt.

**5.** Gerät gemäß Anspruch 4, das zusätzlich Mittel zur Speicherung der umgewandelten Daten bietet.

**6.** Gerät gemäß Anspruch 4 oder Anspruch 5, das zusätzlich Mittel zur Anzeige der umgewandelten Daten bietet.

**Revendications**

**1.** - Procédé de transformation de données de signaux électriques provenant de capteurs à l'aide d'un microprocesseur, comprenant les étapes de :

a) collecte et stockage de données de signaux électriques dans un fichier informatique (2) ;
b) calcul d'un champ vectoriel de densité de courant par résolution du problème inverse linéaire non pondéré apparenté (6) ;
c) traitement du champ vectoriel de densité de courant à l'aide d'une transformation sLORETA (7) ;
d) calcul d'une matrice de pondération diagonale telle que ses entrées sont déterminées par une fonction croissante de façon monotone de leurs valeurs correspondantes dans les sorties (8) de la méthode sLORETA ;
e) calcul du champ vectoriel de densité de courant par résolution du problème inverse linéaire pondéré apparenté (9) ; et
f) stockage des données résultantes dans au moins un fichier informatique (11).

**2.** - Procédé selon la revendication 1, comprenant en outre l'étape d'application d'une technique d'imagerie de données aux données résultantes stockées pour transformer les données sous une forme appropriée pour une représentation visuelle des données (11).

3. - Procédé selon la revendication 2, comprenant en outre l'étape d'affichage des données transformées pour une inspection visuelle.

4. - Appareil de collecte et de transformation de données de signaux électriques, comprenant :

- des capteurs de collecte de signaux électriques ;
- des moyens de stockage de données de signaux électriques ; et
- au moins un microprocesseur ayant un programme informatique configuré pour mettre en oeuvre les étapes de la revendication 1.

5. - Appareil selon la revendication 4, comprenant en outre des moyens de stockage de données transformées.

6. - Appareil selon la revendication 4 ou la revendication 5, comprenant en outre des moyens d'affichage des données transformées.

## Figure 1

1. Arrange electrodes on head for physiological data collection and set-up computer for data collection

2. Collect physiological data into a computer file

3. Pre-processing Necessary? — Yes → 4. Apply pre-processing step

No

5. Determine noise covariances Cn, lead field A, and prior source covariances Cp

6. Compute Current Density Vector Field x (opt) based on related unweighted linear inverse problem

7. Compute s LORETA result, s, based on the current density vector field, x (opt)

8. Compute diagonal weighting matrix, W, based on s

9. Compute Current Density Vector Field, x (opt), by solving the related weighted linear inverse problem

10. Is the number of iterations below a pre-defined limit or is the difference between x (opt) and the x (opt) obtained in the previous iteration above a pre-defined limit? — Yes

No

11. Transform data for computer screen display and / or for output to computer file

## Figure 2

(a)

(b)

Figure 3

(a)

(b)

(c)

CDR
[μA/mm²]
1.16e-006
1e-006
9e-007
8e-007
7e-007
5.82e-007

275.00 ms

1    2    3

(d)

Figure 4

(a)

(b)

(c)

CDR
[F-distributed]
4201

3500

3000

2500
2101

275.00 ms

(d)

Figure 5

(a)

(b)

(c)

CDR
[μA/mm²]
0.0000118
0.0000110
0.0000100
0.0000090
0.0000080
0.0000070
0.0000059

275.00 ms

(d)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIU et al.** Standardized Shrinking LORETA-FOCUSS (SSLOFO): A New Algorithm for Spatio-Temporal EEG Source Reconstruction. *IEEE TRANSACTIONS ON BIOMEDICAL ENGINEERING,* October 2005, vol. 52 (10), 1681-1691
- **YONGXIA ZHOU et al.** Spatiotemporal source localization with new reweighted method: a simulation study. *IEEE NUCLEAR SCIENCE SYMPOSIUM CONFERENCE RECORD. /2003 IEEE NUCLEAR SCIENCE SYMPOSIUM AND MEDICAL IMAGING CONFERENCE,* 19 October 2003, vol. 4, ISBN 978-0-7803-8257-2, 2776-2779
- **DALE A.M. ; SERENO M.I.** Improved Localization of Cortical Activity by Combining EEG and MEG with MRI Cortical Surface Reconstruction: A Linear Approach. *Journal of Cognitive Neuroscience,* 1993, vol. 5, 162-176
- **FUCHS M. ; WAGNER M. ; KÖHLER T. ; WISCHMANN H.A.** Linear and Nonlinear Current Density Reconstructions. *J Clin Neurophysiol,* 1999, vol. 16, 267-295
- **HÄMÄLÄINEN M. ; LIMONLEMI R.** Interpreting magnetic fields of the brain: minimum norm estimates. *Report TKK-F-A559,* 1984
- **KÖHLER T.** Lösungen des bioelektromagnetischen inversen Problems. *PhD-Thesis,* 1998

- **PASCUAL-MARQUI R.D.** Review of methods for solving the EEG inverse problem. *International Journal of Bioelectromagnetism,* 1999, vol. 1, 75-86
- **PASCUAL-MARQUI R.D.** Standardized low resolution brain electromagnetic tomography (sLORETA): technical details. *Methods & Findings in Experimental & Clinical Pharmacology,* 2002, vol. 24D, 5-12
- **PASCUAL-MARQUI R.D. ; MICHEL C.M. ; LEHMANN D.** Low resolution electromagnetic tomography: a new method for localizing electrical activity in the brain. *International Journal of Psychophysiology,* 1994, vol. 18, 49-65
- **SEKIHARA K. ; SAHANI M. ; NAGARAJAN S.S.** Localization bias and spatial resolution of adaptive and non-adaptive spatial filters for MEG source reconstruction. *Neurolmage,* 2005, vol. 25, 1056-1067
- **TARANTOLA A.** Inverse Problem Theory. Elsevier, 1994
- **WAGNER M.** Rekonstruktion Neuronaler Ströme. Shaker Verlag, 1998
- **WAGNER M. ; FUCHS M. ; KASTNER J.** Evaluation of sLORETA in the presence of noise and multiple sources. *Brain Topogr.,* 2004, vol. 16, 277-80